# EUROPEAN PATENT APPLICATION

(11) **EP 1 566 193 A1**
(43) Date of publication of application: **24.08.2005**
(21) Application number: 04075489.7
(22) Date of filing: 18.02.2004
(51) Int. Cl.: A61M 5/158

(54) **Infusion device provided with septum housing**

(71) Applicant: Unomedical A/S, 3540 Lynge (DK)
(72) Inventor: Nielsen, Jens Egebjerg, 4100 Ringsted (DK); Ludvigsen, Morten, 4700 Naestved (DK); Jensen, Soren, 3540 Lynge (DK); Mathiasen, Orla, 4180 Soro (DK)
(74) Representative: Elmeros, Claus

(57) **Abstract**

The invention concerns an infusion device for subcutaneous administering a medication or a therapeutic fluid to a patient. Said infusion device comprises a base element (1), which comprises fluid receiving means for receiving said fluid and fluid communication means for transferring said fluid into a cannula, said base element being provided with at least one recess (20) accommodating a septum (4) being pierceable by a needle. Said septum is secured to said base element by a septum housing (3) in such a way that a fluid transfer volume is formed in said at least one recess between an internal surface of said septum and an inner section of said recess. Said fluid transfer volume is communicating with said fluid communication means, and said septum housing is accommodating a premounted septum. The infusion device is characterized in that the septum housing is fixed and fluid sealed to said base element in said inner section of said recess.

## Description

The present invention relates to an infusion device for subcutaneous administering a medication or a therapeutic fluid to a patient, comprising a base element, which comprises fluid receiving means for receiving said fluid and fluid communication means for transferring said fluid into a cannula, said base element being provided with at least one recess accommodating a septum being pierceable by a needle.

Infusion devices are generally known in the art for delivering a medication or a therapeutic fluid, including insulin to a subcutaneous site in a patient. Such infusion devices commonly comprise a tubular cannula extending into the subcutaneous site of the patient from a housing adapted to receive the desired medication. Said cannula may either be rigid or soft, a rigid usually being a steel cannula although other possibilities exist, and a soft being e.g. a Teflon® cannula, but it is however possible to utilise several other polymer material cannulas.

In order to inject a soft tubular cannula into a patient, an infusion device may be provided with a septum being pierceable by a discardable insertion needle, allowing for a single injection of the insertion needle surrounded by the soft cannula for placement of the soft cannula inside a patient. A soft cannula allows for increased user comfort when moving around wearing such an infusion device.

In order to improve user mobility, the housing of the infusion device may be disconnectably connected to further components of the infusion system, e.g. to a comparatively large insulin pump. The user is accordingly enabled to perform activities which do not allow the presence of such a pump or the like.

In order to provide such disconnectable means and still maintain a fluid tight sealing towards the interior of the housing and the tubular cannula in order to prevent contamination of the injection site, such devices are commonly provided with a self-sealing penetrable septum on either the housing or the disconnectable component and a hollow needle on the other part adapted to penetrate the septum. Upon withdrawal of the needle from the septum, possibly multiple times, this provides a fluid-tight sealing between the housing and the connector means when medication or therapeutic fluid, particularly insulin is delivered to the patient from the external parts of the infusion system.

The manufacture of such an infusion device including one or more septums is rather cumbersome for several reasons. Firstly, the device is relatively small in order to attach to the skin comfortably and consequently the constituting parts of the device are relatively small, which complicates assembly of the device. Secondly, the securing of a septum inside the device involves both fixation to the device and fluid-sealing against the contributing parts of the device abutting the septum. Fixation is important, as an improperly fixed septum, due to the elasticity of the septum material, elastically bends and provides resistance, when a needle is inserted into the septum, and fixation and fluid sealing is important in order to provide a leak tight device. For these reasons the assembly of the device often involves the use of many small parts, which adds to the complicated manufacture.

Different approaches to secure a septum to an infusion device are known. A known and widely used approach is mounting a septum with a generally circular cross section in a fluid-tight and compressed manner between two annular discs or jaws with a smaller cross section than said septum, compressing the septum to a lesser thickness than in its unstressed condition. A drawback is the thus secured compressed septum being subject to fatigue induced fracturing.

US 5,968,011 discloses a septum containing subcutaneous injection set, which set comprises a base element with a hub and a top element rotatably attached to said base element by the use of a flange. In one embodiment a septum is secured inside a cavity in the base element, said septum being fixed by resting against cannula securing means. The injection set is fluid-sealed by ultrasonic welding the septum sides to the abutting sides of the hub. A drawback is the relatively large number of small parts being used to assemble the injection set.

Another known infusion device in US5,257,980 comprises a base element, provided with a large fluid-sealant upper part being secured to a lower part and to the base part. The septum is placed between said base element and said upper part, and the septum is fixed by the introduction of rib parts on the septum as a fixing arrangement against the under side of the upper part. The securing of the septum is thus achieved by the use of relatively large and bulky upper and lower parts, and by the use of especially to each other adapted upper and lower parts, base element and septum, and these provisions add to the cost of manufacturing the infusion device.

For these reasons there is a need for improvement in the infusion devices of the type mentioned above, in particular with respect to simplifying the manufacture of said infusion device comprising at least one septum.

This object is achieved by an infusion device for subcutaneous administering a medication or a therapeutic fluid to a patient, comprising a base element, which comprises fluid receiving means for receiving said fluid and fluid communication means for transferring said fluid into a cannula, said base element being provided with at least one recess accommodating a septum being pierceable by a needle, wherein said septum is secured to said base element by a septum housing in such a way that a fluid transfer volume is formed in said at least one recess between an internal surface of said septum and an inner section of said recess, said fluid transfer volume communicating with said fluid communication means, and said septum housing accommodating a premounted septum, wherein said septum housing is being fixed and fluid sealed to said base element in said inner section of said recess.

The manufacture of said infusion device, particularly the securing, i.e. the fixation and fluid-sealing of the septum to the base element, has been simplified by utilising a base element provided with a recess and a septum premounted in a septum housing, which is inserted in, fixed to and fluid-sealed to the base element in the inner section of said recess. Accordingly, due to the need for a relatively small part of the septum housing abutting the inner section of said recess in said base element in order to fix and fluid-seal said septum housing to said base element, the size of said parts needed for securing said septum to said infusion device can be kept to a minimum. A further advantage by providing a relatively small fixation and fluid-sealing area is decreasing the need for specially formed upper parts or lids adapted to fit a corresponding base element and/or lower part, nor for a specially formed septum provided with grooves etc.

In one embodiment, said fluid transfer volume is accommodating cannula securing means. In combination with the septum securing means a fluid-tight sealing and a passage to the fluid communication means is provided.

In another embodiment the septum housing is a tubular element accommodating said septum, said tubular element having at least one end forming a substantially partial enclosure over one surface of the septum. This allows advantageously for a small septum housing having a non-complex form, further simplifying the manufacture of said septum housing. Further, the only externally exposed part of the septum is the open part of the enclosure, which part may be made optionally small, allowing for lesser possibility for contamination of this exposed part of the septum.

In a further embodiment the septum housing is a cannula bushing accommodating said septum. The advantage is further reducing the number of device parts being used to secure said septum and thus to assemble the infusion device. In another embodiment one of the surfaces of said septum is substantially exposed.

In further embodiments the septum housing is fixed to said base element by welding, in particular ultrasonic welding, or by a snap-lock. Welding is both fixes and fluid-seals the septum housing to the base element in one operation. A snap-lock reduces the need for a welding treatment after insertion of the septum housing into the recess in order to fix said septum housing to the base element.

In a still further embodiment the septum housing is made fluid-sealant by ultrasonic welding it to the base element or by a gasket arranged between said septum housing and the inner section of said recess. Providing a gasket is advantageous in cases in which the insertion is eased by a clearance being provided between the inner section of the recess and the septum housing, said clearance leaving space for a gasket.

In further embodiments the septum is fixed inside said septum housing by friction, or by welding, in particular ultrasonic welding. Friction advantageously utilises the septum material property against the inner sides of the septum housing and need no further operation. Welding is advantageous as it in one operation both fixes and fluid-seals the septum housing to the base element.

The infusion device according to the invention will now be described, by way of example, with reference to the diagrammatic drawings in which
- Fig. 1 is a cross-sectional side view illustrating a prior art infusion device,
- Fig. 2, 3, 4, and 5 are cross-sectional side views illustrating different embodiments of an infusion device according to the present invention, and
- Fig. 6, 7, and 8 are cross-sectional side views illustrating different embodiments.

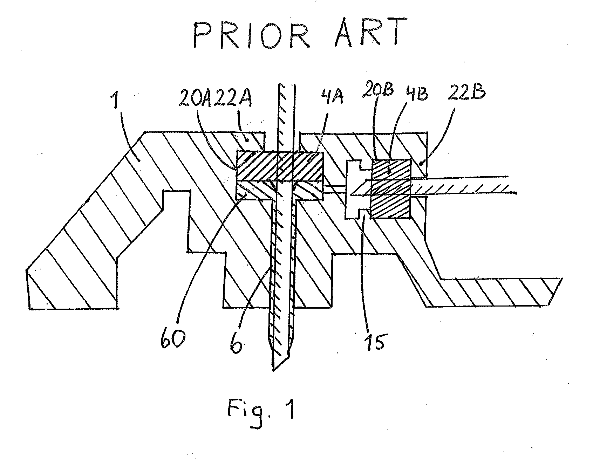

Fig. 1 shows a prior art infusion device, particularly for administering insulin to a patient, comprising a base element 1 having two recesses 20A, 20B to accommodate two septums 4A, 4B, respectively. The first septum 4A is provided in order to fluid-seal the device during and after the injection of a soft cannula 6, which is provided on the infusion device. The second septum 4B is provided in order to fluid-seal the device during and after the insertion of a disconnectable component needle 7 providing connection to further components of the infusion system, e.g. a pump (not shown).

Fixation and fluid-sealing of the septums 4A and 4B is accomplished by covering part of the externally facing surface of the septum 4A, 4B by a covering part 22A, 22B, respectively, of the base element 1, while the septum 4A, 4B, respectively, is resting against cannula securing means 60 provided under the septum 4A or a base element neck 15 being provided for this purpose, and thus by slightly compressing the septum 4.

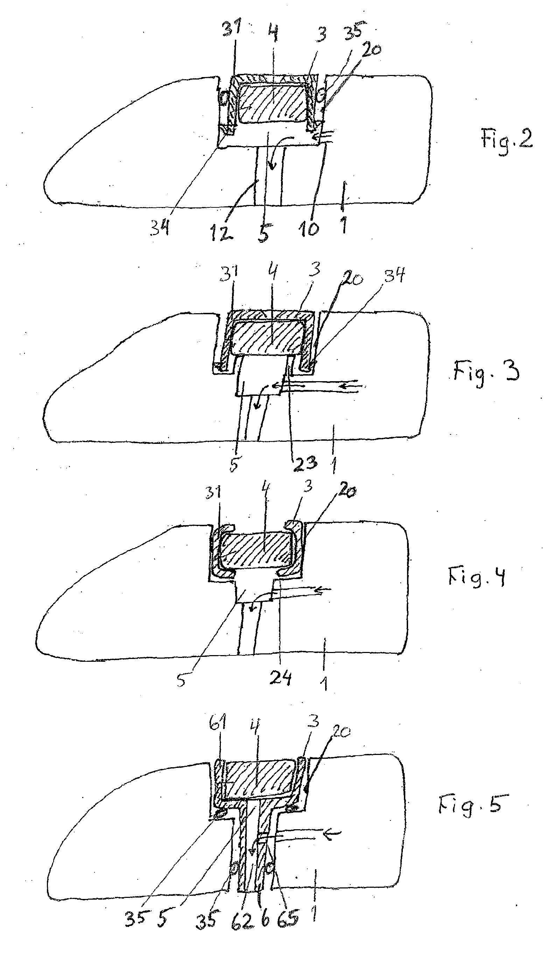

Fig. 2 illustrate one embodiment of the present invention in which said base element 1 comprises a recess 20 accommodating a septum housing 3 including a septum 4. The septum housing 3 is a tubular element 31, one external end of which is formed or bent over one external surface of the septum 4, forming a substantially partial enclosure over one surface of said septum 4 and leaving one of the surfaces of said septum 4 substantially exposed, namely the septum surface facing a fluid transfer volume 5 formed between said surface of the septum 4 and the inner section of the recess 20 in the base element 1. The forming or bending of the tubular element 31 may be accomplished in the moulding operation for the tubular element 31 or during a bending operation after moulding. A septum 4 is provided and pre-mounted in the tubular element 31 by inserting the septum 4 into the housing space formed inside the tubular element 31. The tubular element 31 is fixed to the base element 1 in the inner section of the recess 20 by a snap-lock 34, in this case provided on the opposing end of the tubular element 31 as compared to the formed or bend end of the same. Said snap-lock 34 is either a projection or slot provided on said tubular element 3, which projection or slot is engaging into a corresponding slot or projection, respectively, provided on the base element 1 in the inner section of the recess 20.

In Fig. 2, a gasket 35 is provided for the fluid-sealing of the tubular element 31 to the base element 1, said gasket 35 being provided between a clearance between said tubular element 31 and said base element, said clearance being located in the recess 20.

Cannula securing means may be provided inside the infusion device according to the invention inside the fluid transfer volume 5, similar to the arrangement of the prior art infusion device of Fig. 1.

Fluid-sealing of the septum 4 is provided by ultrasonic welding of the septum 4 to the tubular element 31, this may be performed during pre-mounting of the septum 4 or after insertion of the tubular element 31 into the recess 20, and by ultrasonically welding the septum 4 to the base element 1 by the provision of a septum resting flange 23 in the inner section of the recess 20 in the base element 1.

In Fig. 3, the fixation of the septum 4 is performed essentially as stated above for Fig. 2, but in this case fixation and fluid-sealing is accomplished by in the inner section of the recess 20 providing a neck or flange 23 for ultrasonically welding the septum 4 to said neck or flange 23. A gasket may then be dispensed with.

In Fig. 4, which shows yet another embodiment of the present invention, said septum housing 3 comprises a tubular element 31, both ends of which are bent or formed over respective ends of the septum 4. The septum 4 is fixed to the tubular element 31 either by bending of the ends of the tubular element 31 or by pre-mounting said septum 4 into the tubular element 31 provided with formed or bent ends, taking advantage of the flexible material of the septum 4. The fixation of the tubular element 31 containing the septum 4 is performed by ultrasonically welding the tubular element 31 to the interior section of the recess in said base element 1. Fluid-sealing is provided by ultrasonic welding of the septum 4 to the tubular element 31, either before or after insertion of said tubular element 31 into said recess 20. In order to secure said fixation of said septum housing 4 inside the inner section of the recess 20 a neck 24 of the inner section of said recess is provided as a rest for the tubular element 31.

In fig. 5, another embodiment of the present invention is shown, in which the septum housing 3 comprises the cannula bushing 61, this being of a size to accommodate the septum 3 inside at least one part of said cannula bushing 61. In Fig. 5, said cannula bushing 61 is extending from the soft cannula 6 through the base element 1 and to the external surface of the base element 1, and is expanded in the end part extending inside the inner section of the recess 20. The fluid transfer volume 5 comprises in this embodiment the cannula interior 62. Fixation of the septum 4 to the base element 1 is accomplished by pre-mounting the septum 4 in said cannula bushing 61, said bushing advantageously formed in one piece with the soft cannula 6, and by inserting said cannula 6 with cannula bushing 61 containing the septum 4 into the recess 20. Fluid-sealing is provided by the use of one or two gaskets 35 provided in the inner section of the recess 20 and around the cannula 6. As seen in Fig. 5, a small passage opening 65 is provided in the cannula bushing 61 for the medication or therapeutic fluid to pass into said cannula passage 62 from the fluid communication means.

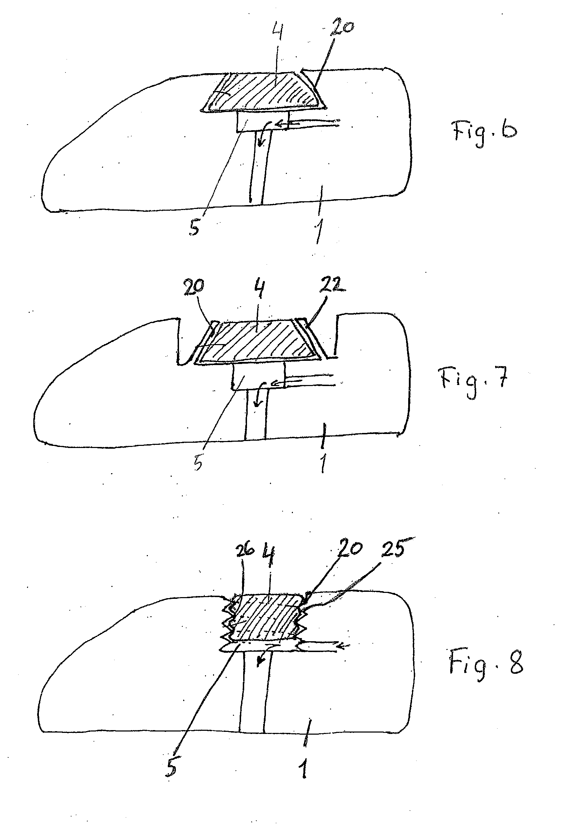

In Fig. 6, the recess 20 in the base element 1 has a frustroconical shape, accommodating a septum 4 being of substantially the same shape and size as the recess 20. Insertion of the septum is accomplished by pressing the septum into the recess 20, the material property of the septum 4 allowing this. Fluid-sealing is accomplished by ultrasonic welding of the septum 4 to the inner section of the recess 20.

In Fig. 7, the base element 1 comprises a tubular flange 22, which when formed or bent over the septum 4 provides a frustroconical recess accommodating said septum 4.

In Fig. 6 and 7, the septum 4 may initially be of either a cylindrical shape or a frustroconical shape with more or less angle in respect to the axis of rotation of the septum 4. This way, the fixation of the septum 4 inside the recess 20 to the base element 1 is supported by the flexible material property of the septum, exerting a force on the sides of the recess 20, if the septum 4 has a slightly smaller angle in respect to the axis of rotation initially than does the recess 20.

In Fig. 8, the base element 1 comprises first threads 25 provided on said base element 1 in the inner section of said recess 20, said first threads 25 engaging with second threads 26 provided on the septum 4. Fluid sealing may be provided by ultrasonic welding.

The septum material is preferably a pierceable flexible material, often rubber or other material, and the base element and septum housing material may be any kind of suitable material, preferably plastic.

The size and shape of the septum, septum housing, and recess are adapted to each other, and may be any of these, preferably comprising a substantially cylindrical septum. Optionally, a previously slit septum may be utilised in order to facilitate the passage of a blunt needle, e.g. as is described in US 6,017,328 to Unomedical.

In fig. 2 to 5 are shown embodiments of the invention with only one recess and one septum, but obviously the infusion device according to the present invention may be conceived comprising two or more recesses 20 accommodating septum housings 3 and septums 4, respectively.

## Claims

1. Infusion device for subcutaneous administering a medication or a therapeutic fluid to a patient, comprising
a base element, which comprises
fluid receiving means for receiving said fluid and
fluid communication means for transferring said fluid into a cannula,
said base element being provided with at least one recess accommodating a septum being pierceable by a needle,
wherein said septum is secured to said base element by a septum housing in such a way that a fluid transfer volume is formed in said at least one recess between an internal surface of said septum and an inner section of said recess, said fluid transfer volume communicating with said fluid communication means, and
said septum housing accommodating a premounted septum,
**characterized by**
said septum housing being fixed and fluid sealed to said base element in said inner section of said recess.

2. Infusion device according to claim 1, wherein said fluid transfer volume is accommodating cannula securing means.

3. Infusion device according to claim 1 or 2, wherein said septum housing is a tubular element accommodating said septum, at least one end of which tubular element forming a substantially partial enclosure over one surface of said septum.

4. Infusion device according to any of the preceding claims, wherein said septum housing is a cannula bushing.

5. Infusion device according to any of the preceding claims, wherein one of the surfaces of said septum is substantially exposed.

6. Infusion device according to any of the preceding claims, wherein said septum housing is fixed to said base element by welding.

7. Infusion device according to claim 6, wherein said septum housing is fixed to said base element by ultrasonic welding.

8. Infusion device according to any of the claims 1-5, wherein said septum housing is fixed to said base element by a snap-lock.

9. Infusion device according to any of the preceding claims, wherein said septum housing is fluid-sealed by ultrasonic welding to said base element.

10. Infusion device according to any of claims 1-8, wherein said septum housing is fluid-sealed by a gasket arranged between said septum housing and said inner section of said recess.

11. Infusion device according to any of the preceding claims, wherein said septum is fixed inside said septum housing by friction.

12. Infusion device according to any of claims 1-10, wherein said septum is fixed inside said septum housing by welding.

13. Infusion device according to claim 12, wherein said septum is fixed inside said septum housing by ultrasonic welding.
